(19) Europäisches Patentamt
European Patent Office
Office européen des brevets

(11) **EP 3 156 091 B1**

(12) **EUROPÄISCHE PATENTSCHRIFT**

(45) Veröffentlichungstag und Bekanntmachung des Hinweises auf die Patenterteilung:
**26.02.2020 Patentblatt 2020/09**

(51) Int Cl.:
*A61M 16/00* *(2006.01)*          *A61M 39/10* *(2006.01)*

(21) Anmeldenummer: **16002136.6**

(22) Anmeldetag: **05.10.2016**

(54) **VORRICHTUNG ZUR ÜBERWACHUNG EINER DISKONNEKTION**

DEVICE FOR MONITORING A DISCONNECTION

DISPOSITIF DE SURVEILLANCE D'UNE DÉCONNEXION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.10.2015 DE 102015012930**

(43) Veröffentlichungstag der Anmeldung:
**19.04.2017 Patentblatt 2017/16**

(73) Patentinhaber: **Löwenstein Medical Technology S.A.**
**2557 Luxembourg (LU)**

(72) Erfinder:
• **Adametz, Benjamin**
**22609 Hamburg (DE)**

• **Tiemann, Björn**
**22359 Hamburg (DE)**

(74) Vertreter: **Marx, Thomas**
**Löwenstein Medical Technology GmbH + Co. KG**
**IP Management**
**Kronsaalsweg 40**
**22525 Hamburg (DE)**

(56) Entgegenhaltungen:
| | |
|---|---|
| **EP-A1- 0 968 020** | **WO-A1-2014/030098** |
| **US-A1- 2004 016 431** | **US-A1- 2006 086 357** |
| **US-A1- 2010 071 696** | **US-A1- 2015 144 130** |

**Beschreibung**

Die vorliegende Erfindung bezieht sich auf die Erkennung von Fehlern bei Beatmungssystemen und eine entsprechende Alarmierung bei Fehlern.

Bei der Beatmung bilden das Beatmungsgerät und das respiratorische System des Patienten ein miteinander gekoppeltes pneumatisches System. Über das Patienteninterface-System, welches zumindest aus dem Beatmungsschlauch und dem Patienteninterface (beispielsweise Endotrachealtubus oder Maske) besteht, sind das Beatmungsgerät und das respiratorische System pneumatisch miteinander verbunden.

Die Diskonnektion (Unterbrechung der Verbindung) im Patienteninterface-System ist ein ungewollter Zustand, bei dem eine teilweise oder vollständige pneumatische Entkopplung von Teilen des Patienteninterface-Systems, bestehend beispielsweise aus dem Beatmungsgerät, dem Beatmungsschlauch und einer Beatmungsmaske, erfolgt. Bedingt durch die Diskonnektion wird der Patient nicht mehr oder nicht mehr adäquat beatmet.

Es besteht für den Arzt daher die Notwendigkeit, eine Diskonnektion zu erkennen. Eine Detektion und Alarmierung einer Diskonnektion ist Stand der Technik.

Die Detektion kann über Druck- oder Flowmessung erfolgen. Bei einer ausschließlich gerätenahen Druckmessung oder bei der Verwendung von zusätzlichen Komponenten wie Filter oder Konnektionsschlauch ("Gänsegurgel") zwischen Ausatemsystem und Patient kann diese Erkennung einer Diskonnektion nicht über das Drucksignal durch das Gerät erfolgen. Bei Verwendung des Flowsignals zur Detektion kann die Detektionsschwelle je nach den angeschlossenen Komponenten im Atemstrom sehr unterschiedlich liegen. Bei einer festen Detektionsschwelle kommt es zu Fehlalarmen oder zu fehlender Sicherheit.

Die US2004/0016431 A1 beschreibt die Erkennung einer Diskonnektion auf Basis von Druck und Flow und der Compliance des Patienten während der Beatmung.

Die US2006/0086357 A1 beschreibt die Erkennung einer Diskonnektion auf Basis eines zweistufigen Algorithmus mit hoher und niedriger Sensitivität während der Beatmung.

Die EP0968020 A1 beschreibt die Erkennung von Diskonnektion und Okklusion bei Doppelschlauchsystemen.

Die WO2014/030098 A1 beschreibt die Erkennung von Diskonnektion bei Einschlauchsystemen mit hohen Widerständen anhand von Methoden zur Korrelation von Druck und Fluss und Compliance des Patienten.

Die US2015/144130 A1 beschreibt die Erkennung von Diskonnektion und Okklusion auf Basis von Fluss und Motordrehzahl.

Die US2010/0071696 A1 beschreibt die Erkennung der Leckage und der Atmungsanstrengung des Patienten.

Die Alarmgrenzwerte können aber bei den bekannten Geräten nicht empfindlich genug eingestellt werden, um etwa einen Alarm möglichst rechtzeitig auszulösen, da dies die bereits jetzt unbefriedigend hohe Fehlalarmrate weiter erhöhen würde.

Aufgabenstellung

Der vorliegenden Erfindung liegt daher das technische Problem zugrunde, eine Vorrichtung zur Überwachung einer Diskonnektion anzugeben, die bei sehr geringer Fehlalarmrate rechtzeitig und zuverlässig Alarm auslöst. Zur Lösung dieses Problems wird erfindungsgemäß eine Vorrichtung gemäß Anspruch 1 und ein Verfahren gemäß Anspruch 7 vorgeschlagen. Auf diese Weise kann eine Diskonnektion von einer Beatmungskurve unterschieden werden.

Vorteilhaft umfasst die Vorrichtung einen Fluss-Sensor und/ odereinen Druck-Sensor. Alternativ ermittelt die Vorrichtung den Fluss und/oder Druck aus der Drehzahl und/oder Leistungsaufnahme des Beatmungsgebläses.

Ergänzend oder alternativ umfasst die Vorrichtung einen $CO_2$-Sensor. Dieser $CO_2$-Sensor erfasst beispielsweise das endtidale Kohlenstoffdioxid (et $CO_2$) oder den $CO_2$-Gehalt durch eine transkutane Messung.

Ergänzend oder alternativ ist ein $CO_2$-Sensor mit der Vorrichtung koppelbar. Dieser $CO_2$-Sensor erfasst beispielsweise das endtidale Kohlenstoffdioxid (et$CO_2$) oder den $CO_2$-Gehalt durch eine transkutane Messung.

Eine adaptive Anpassung des Alarms ist möglich, wenn der Zeitraum und/oder der Grenzwert einstellbar sind.

Der Zeitraum und/oder der Grenzwert können auch im Gerät hinterlegt sein oder fortlaufend ermittelt werden.

Vorteilhaft wird der Grenzwert unter Anwendung der Bewegungsgleichung oder davon abgeleiteter Gleichungen und unter Berücksichtigung von Werten, die indikativ für den zeitlichen Verlauf des Atemgasstromes (25) sind, ermittelt. Auf diese Weise kann eine Diskonnektion von einer Beatmungskurve unterschieden werden.

Erfindungsgemäß ist auch vorgesehen, dass die Einstellung von Zeitraum und/oder Grenzwert automatisch beispielsweise anhand von Daten des Patienteninterfacesystems und/oder über eine Anwenderschnittstelle durch den Anwender erfolgt. Die Anwenderschnittstelle kann ein Touchscreen sein oder ein Display welches ein, über einen Encoder, ansteuerbares Auswahlfeld darstellt.

Erfindungsgemäß sind die Grenzwerte Compliance (C) Werte.

Vorteilhaft ist zudem eine Ausgabeeinheit zur Darstellung der Messwerte vorgesehen. Beispielsweise kann die Ausgabeeinheit als ein Touchscreen Display ausgebildet sein.

Ergänzend oder alternativ ist eine Darstellung und Auswertung des Triggers vorgesehen.

Gegenstand der vorliegenden Erfindung ist auch ein Verfahren zur Überwachung der Diskonnektion eines Patienteninterfacesystems während der Beatmung bei dem Werte die indikativ für den zeitlichen Verlauf des Atemgasstromes sind ermittelt werden und einer Datenauswertung unterzogen werden, wobei basierend auf der Datenauswertung ein Alarm auslöst wird, wenn zumindest ein Wert der indikativ für den zeitlichen Verlauf des Atemgasstromes ist für einen bestimmten Zeitraum von einem bestimmten Grenzwert abweicht. Auf diese Weise kann eine Diskonnektion von einer Beatmungskurve unterschieden werden.

Das Verfahren ist bevorzugt auch dadurch gekennzeichnet, dass der Zeitraum und/oder der Grenzwert einstellbar sind.

Das Verfahren ist bevorzugt auch dadurch gekennzeichnet, dass der Zeitraum und/oder der Grenzwert auch im Gerät hinterlegt sein können oder fortlaufend ermittelt werden.

Vorteilhaft wird der Grenzwert unter Anwendung der Bewegungsgleichung oder davon abgeleiteter Gleichungen und unter Berücksichtigung von Werten, die indikativ für den zeitlichen Verlauf des Atemgasstromes (25) sind, ermittelt. Auf diese Weise kann eine Diskonnektion von einer Beatmungskurve unterschieden werden.

Das Verfahren ist auch bevorzugt auch dadurch gekennzeichnet, dass die Einstellung von Zeitraum und/oder Grenzwert automatisch beispielsweise anhand von Daten des Patienteninterfacesystems und/oder über eine Anwenderschnittstelle durch den Anwender erfolgt.

Bei dem erfindungsgemäßen Verfahren ist auch vorgesehen, dass zumindest ein Messwert angezeigt wird und über die Anwenderschnittstelle zumindest ein Grenzwert für zumindest einen Messwert des Atemgasstromes und eine Zeitdauer für den Grenzwert vorgebbar ist.

Gegenstand der vorliegenden Erfindung ist auch eine Steuerung, die Elemente zur Umsetzung des Verfahrens aufweist.

Erfindungsgemäß ist vorgesehen, dass das Atemgas vom Gerät mit angeschlossenem Patienteninterface-System frei, also ohne Patienten, abströmt und das Gerät die dabei ermittelten Druck und/oder Flusswerte speichert und als Grenzwerte für die Ermittlung einer Diskonnektion berücksichtigt.

Der Anwender kann einen Prozent -Wert oder einen Bruchteil dieses Abströmwertes als Grenzwert vorgeben. Es ist auch vorgesehen, dass das Gerät automatisch einen ProzentWert oder einen Bruchteil dieses Abströmwertes als Grenzwert speichert und anwendet.

Erfindungsgemäß können ein Fluss-Sensor und ein Druck-Sensor vorgesehen sein, deren Messwerte beide für die Ermittlung einer Diskonnektion berücksichtigt werden. Durch diese Messwertredundanz wird die Sicherheit der erfindungsgemäßen Vorrichtung erhöht. Durch diese Redundanz lässt auch sich die Fehlalarmquote weiter senken.

Vorteilhaft löst die Datenauswerteeinrichtung einen Alarm nur dann aus, wenn die beiden ermittelten Werte für eine bestimmte Zeit unter einem bestimmten Grenzwert liegen.

Da kurzzeitige, einmalige Abfälle von Druck oder Fluss in einem Zeitraum bis zu 2 Sekunden keine Gefahr darstellen, beträgt der bei der erfindungsgemäßen Vorrichtung einstellbare Grenzwert bevorzugt beispielsweise zwischen 75 und 85% vom ermittelten Wert. Der Alarm wird ausgelöst, wenn die Unterschreitung dieses Grenzwertes einen kritischen Zeitraum überschreitet, der vorteilhaft beispielsweise zwischen 3 und 30 Sekunden, vorzugsweise zwischen 5 und 10 Sekunden beträgt.

Alle für den Einzelfall notwendigen Werte werden vorteilhaft kontinuierlich in einem rollierenden Speicher mit beispielsweise zumindest 30 Minuten Länge zwischengespeichert. Im Fall eines Alarms werden die Daten der letzten 30 Minuten in einen festen Speicher, beispielsweise eine Festplatte, eine Memory Card oder ähnliches übertragen. Die dem Alarm folgenden Daten werden noch für eine gewisse weitere Zeit (beispielsweise für etwa 5 Minuten) in den Festspeicher geschrieben, damit dem Arzt die Zeitintervalle vor und nach dem Alarm zur Diagnose zur Verfügung stehen.

Vorteilhaft weist die erfindungsgemäße Vorrichtung auch ein Display auf, auf dem die gemessenen Werte angezeigt werden. Um größere Sprünge zu verhindern, werden die Werte beispielsweise über 2 Sekunden gemittelt und dann erst angezeigt. Ein Alarm wird auf jeden Fall akustisch und bevorzugt auch optisch angezeigt. Darüber hinaus können untergeordnete Alarmmeldungen vorgesehen sein, die beispielsweise ausgelöst werden, wenn sich die Messwerte um mehr als einen einstellbaren Toleranzwert unterscheiden (etwa um mehr als 3%), oder wenn die Batteriespannung zu niedrig ist oder eine andere Systemfehlfunktion auftritt. Dabei ist wesentlich, dass sich die untergeordneten Systemalarme deutlich von einem Alarm unterscheiden.

Vorteilhaft kann in einem Auswahlmenü des Displays eine Auswahl des Patienteninterface erfolgen. Je nach gewähltem Patienteninterface variieren die Grenzwerte für die Auslösung des Diskonnektionsalarms.

Der Diskonnektionsalarm bietet beispielsweise die Möglichkeit zur individuellen Feineinstellung. Dies erfolgt durch den Anwender beispielsweise in einem Auswahlmenü des Displays als zulässige Diskonnektion in Prozent (%). Dabei ist 1 % die niedrigste Grenzwert und 99 % der höchste Grenzwert. Bevorzugt sind Werte im Bereich zwischen 1 % und 99% einstellbar. Beispielsweise in gerasteten Stufen von 2% oder 5%.

Der Diskonnektionsalarm kann bevorzugt beispielsweise bei folgenden Konfigurationen deaktiviert werden. Wenn der Anwender als Patienteninterface eine Maske wählt und ein Leckageschlauchsystem einsetzt. Wenn der Anwender als Patienteninterface ein Mundstück wählt.

Erfindungsgemäß können beispielsweise durch die Einstellung der zulässigen Diskonnektion und der Alarmaktivierungszeit Diskonnektionen zuverlässig entdeckt werden.

Es können auch Voreinstellungen für die zulässige Diskonnektion und/oder die Alarmaktivierungszeit vorgesehen sein, die entsprechend dem Patienteninterface und/oder dem Schlauchtyp und/oder entsprechend den Therapieeinstellungen Werte für die zulässige Diskonnektion und/oder die Aktivierungszeit vorschlagen oder anwenden.

Erfindungsgemäß kann auch die Aktivierungszeit eingestellt werden. Das ist der Zeitraum, der zur Alarmauslösung benötigt wird, nachdem eine Diskonnektion erkannt wurde. Der einstellbare Zeitraum variiert je nach Patienteninterface und Patienten. Bei einem Erwachsenen sind für das Patienteninterface Maske oder invasiver Zugang beispielsweise 2 - 70 Sekunden einstellbar. Bei einem Erwachsenen sind für das Patienteninterface Mundstück 2 - 700 Sekunden einstellbar. Bei einem Kind sind für das Patienteninterface Maske oder invasiver Zugang oder Mundstück beispielsweise 2 - 35 Sekunden einstellbar.

Die Einstellung der Aktivierungszeit wird dem Anwender am Display dargestellt. Im Falle eines Touchscreens ist die Aktivierungszeit auch am Display einstellbar.

Vorteilhaft weist die erfindungsgemäße Vorrichtung auch eine Schnittstelle auf, mit dem Alarmdaten direkt oder per Modem an einen lokalen Rechner oder an einen dezentralen Auswerterechner in einer Klinik oder bei einem niedergelassenen Arzt übertragen werden können.

Für den Arzt ist die Kenntnis des Zeitpunktes eines Alarms wichtig. Beispielsweise um feststellen zu können, ob Risikohäufungen zu bestimmten Zeitpunkten und/oder im Zusammenhang mit bestimmten Ereignissen auftreten. Die Mitverwendung einer Echtzeituhr und die Aufzeichnung der Echtzeit in Zusammenhang mit Alarmereignissen sind daher vorteilhaft.

Beispielsweise protokolliert die Vorrichtung daher die Alarme und gibt sie auf Anwenderauswahl über die Schnittstelle aus oder zeigt sie am Display an.

Ausführungsbeispiel

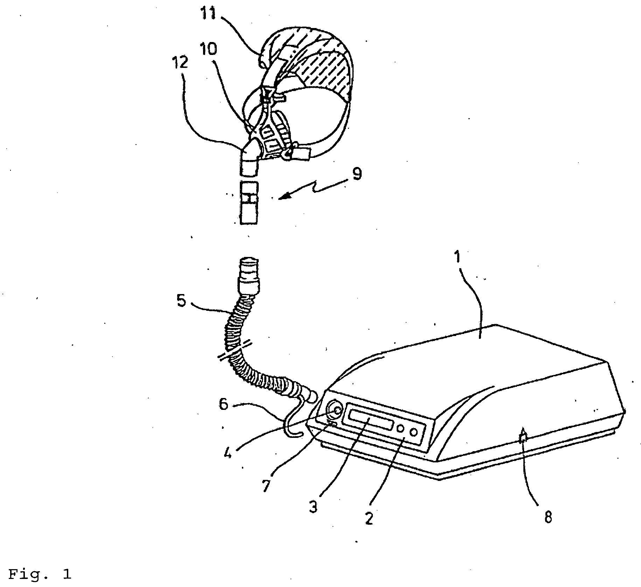

Fig. 1 zeigt den grundsätzlichen Aufbau einer Vorrichtung zur Beatmung. Im Bereich des Beatmungsgerätes (1) mit Bedienfeld (2) sowie Anzeige (3) ist in einem Geräteinnenraum ein Elektromotor (13, nicht dargestellt) mit Gebläse angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf. Im Bereich einer, dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

Der Motor (13) des Beatmungsgerätes ist als ein mehrphasiger Motor realisiert. Als Motor können bürsten- und sensorlose Gleichstrommotoren sowie Synchronmotoren verwendet werden. Im Bereich des Gerätegehäuses befindet sich ein User-Interface (14) für die Anwenderinformation und / oder Anwendersteuerung. In einem Geräteinnenraum ist eine Atemgaspumpe angeordnet, die als Elektro-Motor mit Lüfterrad (13) ausgeführt ist, dessen Betrieb über eine Motorsteuerung (15) regelbar ist. Der Betrieb des Motors und dessen Leistungsregelung sind durch die Motorsteuerung regelbar. Die Motorsteuerung berücksichtigt Daten von zumindest einer Sensoreinrichtung. Die Sensoreinrichtung (19) ermittelt zumindest ein mit dem Atemgasstrom in Zusammenhang stehendes Signal. Beispielsweise kann die Sensoreinrichtung als Fluss-Sensor (19a) und/oder als Druck-Sensor (19b) ausgebildet sein. Ein Analysator ermittelt aus dem mit dem Atemgasstrom (25) in Zusammenhang stehenden Signal Inspirationsphasen und Exspirationsphasen. In zumindest einem Betriebszustand regelt die Motorsteuerung (15) die Lüfterraddrehzahl in Abhängigkeit der ermittelten Atemphase derart, dass während der Inspirationsphase ein im Wesentlichen konstanter positiven Druck aufrechterhalten wird. Der Motor ist derart ausgelegt, dass durch Drehzahländerung ein Druckbereich von beispielsweise 0 bis 80 mbar einstellbar ist. Druckänderungen werden durch Drehzahländerungen des Lüfterrades realisiert.

Die Vorrichtung zur Überwachung der Diskonnektion eines Patienteninterfacesystems (20) - welches bestehen kann aus Verbindungsschlauch (5), Ausatmungselement / Ausatemventil (9), Beatmungsmaske / Patienteninterface (10), Verbindungsschlauch (5), Kupplungselement (12), Bakterienfilter (21), Anfeuchter (22), Gänsegurgel (23) - von einem Beatmungsgerät (1), weist eine Sensoreinrichtung (19) zur Ermittlung von Werten des Atemgasstromes (25) auf, deren Ausgangssignale einer Einrichtung zur Datenauswertung (30) eingespeist werden, die wiederum einen Alarmgeber (40) steuert, wobei die Datenauswertungseinrichtung (30) einen Alarm an dem Alarmgeber (40) auslöst, wenn zumindest ein Wert des Atemgasstromes (25) für einen bestimmten Zeitraum (50) außerhalb einem bestimmten Grenzwert (60) liegt. Die Sensoreinrichtung (19) kann einen Fluss-Sensor (19a) und/oder einen Druck-Sensor (19b) umfassen. Der Zeitraum (50) und/oder der Grenzwert (60) sind beispielsweise einstellbar. Die Einstellung erfolgt automatisch, beispielsweise anhand von Daten des Patienteninterfacesystems und/oder die Einstellung erfolgt über eine Anwenderschnittstelle (14) durch den Anwender. Zudem ist eine Ausgabeeinheit (27) zur Darstellung der Messwerte (26) vorgesehen.

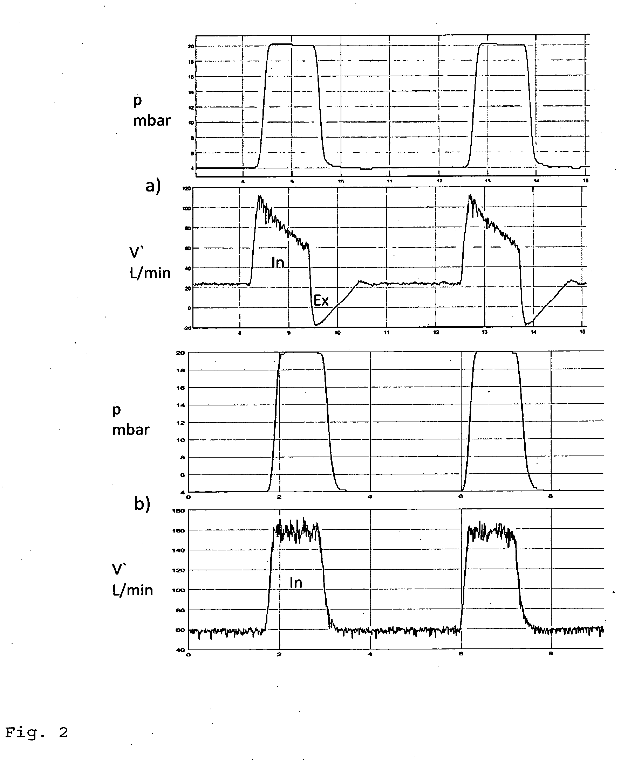

Fig. 2 zeigt Druck- und Flusskurven bei der Beatmung. In Fig. 2a erkennt man oben, wie sich ein voreingestellter (inspiratorischer) Druck (p) für die Beatmung einstellt. In der unteren Darstellung sieht man den resultierenden Fluss (V'). Dieser steigt mit dem Druckhub in der Inspiration (In) an und fällt bei nachlassendem Druck in der Exspiration (Ex) unter das Ausgangsniveau. Dieses ist bedingt durch den (negativen) Ausatemstrom.

Anerkannt ist, dass sich die Zusammenhänge der Beatmungsgrößen Druck (p), Compliance (C), Volumen (V), Fluss (V') und Resistance (R) durch die Bewegungsgleichung beschreiben lassen:

$$\Delta P = (1/C) * V + R * V´ \quad (80)$$

In Fig. 2b ist die Situation aus Fig. 2a mit einer Diskonnektion im Patienteninterfacesystem dargestellt. Man erkennt oben, wie sich ein voreingestellter (inspiratorischer) Druck (p) für die Beatmung einstellt. In der unteren Darstellung sieht man den resultierenden Fluss (V'). Dieser steigt mit dem Druckhub in der Inspiration (In) an und fällt bei nachlassendem Druck in der Exspiration (Ex) unter das Ausgangsniveau. Allerdings ist der Verlauf deutlich verschieden von dem Flussverlauf aus Fig. 2a. Ein wesentlicher Unterschied ist der höhere Fluss bei Diskonnektion (etwa 160 L/min) im Vergleich zur normalen Beatmung mit etwa 115 L/min und der fehlende Ausatemstrom bei Diskonnektion. Je nachdem wo die Diskonnektion vorliegt und wieviel Leckagefluss im Bereich der Diskonnektion vorliegt, können der inspiratorische Fluss und der exspiratorische Fluss in charakteristischer Weise verändert sein. Dies ergibt sich mathematisch aus der Bewegungsgleichung; im Falle einer Diskonnektion wird die Compliance (C) groß.

Grundsätzlich gilt, dass bei Vorhandensein eines Gasflusses die dynamische Atemmechanik aus der Bewegungsgleichung ermittelt werden kann. Dies eröffnet beispielsweise die Möglichkeit der simultanen Bestimmung von Compliance und Resistance. Alternativ sind auch bei einem gegebenen oder gemessenen Druck die zu erwartenden Flussverläufe bestimmbar. Wenn Druck und Fluss gemessen werden, kann somit aus dem zeitlichen Verlauf auf das Vorhandensein einer Diskonnektion geschlossen werden.

Hierzu sind verschiedene Methoden denkbar:
Die multiple lineare Regressionsanalyse (Uhl RR, Lewis FJ. Digital computer calculation of human pulmonary mechanics using a least squares fit technique. Comput Biomed Res. 1974 Oct;7(5):489-95.) ist eine Vielpunkt-Methode und basiert auf dem durch die Bewegungsgleichung beschriebenen linearen RC-Modell.

Die Anwendung der multiplen linearen Regressionsanalyse setzt eine digitale Signalverarbeitung voraus. Ein digitales Messsystem erfasst die respiratorischen Messdaten, Atemwegsdruck (Paw), Atemgasvolumen (V) und Atemgasfluss (V') mit einer gewissen Abtastfrequenz, d.h. zu definierten Zeitpunkten t1, t2, ... tn liest das Messsystem Paw-V-V' Wertetripel ein. Für jedes Wertetripel wird die Bewegungsgleichung formuliert. Dabei entsteht folgendes Gleichungssystem:

$$Paw(t1) = V(t1)/C + V´(t1) * R$$

$$Paw(t2) = V(t2)/C + V´(t2) * R$$

$$Paw(t3) = V(t3)/C + V´(t3) * R$$

$$Paw(tn) = V(tn)/C + V´(tn) * R$$

Die Abtastrate bestimmt die Anzahl der pro Atemzug erfassten Messpunkte und damit auch die Größe des Gleichungssystems, aus dem die Variablen atemzugsweise bestimmt werden können.

Bei der Slice Methode (Guttmann J, Eberhard L, Fabry B, u. a. Determination of volume-dependent respiratory system mechanics in mechanically ventilated patients using the new SLICE method. Technol Health Care.1994;2:175-91) wird die multiple lineare Regressionsanalyse innerhalb eines einzelnen Atemzuges mehrfach angewendet. Hierzu wird das Atemzugsvolumen in 6 gleich große Volumenabschnitte ("slices") unterteilt. Für jeden Abschnitt werden mittels multipler linearer Regressionsanalyse (least squares fit) jeweils ein Wert für Compliance und Resistance bestimmt. So kann ein intratidaler Verlauf von Compliance und Resistance für jeden Atemzug bestimmt werden.

Alternativ kann die Compliance auch computergestützt durch lineare Regression mit der Methode der kleinsten Quadrate (least squares fit) berechnet werden. Hierbei wird die Bewegungsgleichung für alle Punkte eines Atemzuges gelöst und diejenige Kombination der Einflussgrößen (R, C) ausgewählt, mit welcher die tatsächlichen Messwerte mit dem geringsten quadratischen Fehler reproduziert werden können. Diese Methode hat den Vorteil, dass alle Datenpunkte eines Atemzuges in die Berechnung mit einfließen und dadurch der mögliche Fehler minimiert wird.

Erfindungsgemäß sind auch andere Methoden vorgesehen, die es erlauben aus zumindest den Werten für Druck und Fluss auf das Vorhandensein einer Diskonnektion zu schließen.

Die Vorrichtung zur Überwachung der Diskonnektion eines Patienteninterfacesystems (20) weist dazu eine Einrichtung (19) zur Ermittlung von Werten die indikativ für den zeitlichen Verlauf des Atemgasstromes (25) sind auf, deren Ausgangssignale einer Einrichtung zur Datenauswertung (30) eingespeist werden, die wiederum einen Alarmgeber (40) steuert, wobei die Datenauswertungseinrichtung (30) einen Alarm an dem Alarmgeber (40) auslöst, wenn zumindest ein Wert der indikativ für den zeitlichen Verlauf des Atemgasstromes ist für einen bestimmten Zeitraum (50) von einem bestimmten Grenzwert (60) abweicht. Der Grenzwert ergibt sich hier beispielsweise aus der Bestimmung eines idealisierten Verlaufes im Vergleich zum ermittelten, tatsächlichen Flussverlauf aus der Anwendung der Bewegungsgleichung. Der Grenzwert kann hinterlegt sein oder auch beispielsweise fortlaufend neu ermittelt werden. Ein Beispiel für einen Grenzwert ist die Compliance, die bei einer Diskonnektion ansteigt. Ebenso kann der Druck, der bei einer Diskonnektion abfällt, ein Grenzwert sein. Durch Anwendung der Bewegungsgleichung oder davon abgeleiteter Gleichungen kann ein Wert der indikativ für den zeitlichen Verlauf des Atemgasstromes ist, ermittelt werden. Ebenso kann ein Grenzwert bestimmt werden. Aus einer Veränderung des indikativen Wertes über den Grenzwert hinaus, kann auf eine Diskonnektion geschlossen werden.

Das Verfahren zur Überwachung der Diskonnektion eines Patienteninterfacesystems von einem Beatmungsgerät ermittelt, beispielsweise unter Anwendung der Bewegungsgleichung, Werte die indikativ für den zeitlichen Verlauf des Atemgasstromes sind und unterzieht diese einer Datenauswertung, wobei basierend auf der Datenauswertung ein Alarm auslöst wird, wenn zumindest ein Wert der indikativ für den zeitlichen Verlauf des Atemgasstromes ist für einen bestimmten Zeitraum von einem bestimmten Grenzwert abweicht. Der Grenzwert ergibt sich hier beispielsweise aus der Bestimmung eines idealisierten Verlaufes im Vergleich zum ermittelten, tatsächlichen Flussverlauf aus der Anwendung der Bewegungsgleichung. Auf diese Weise kann eine Diskonnektion von einer Beatmungskurve unterschieden werden.

Bezugsziffern:

| 1 | Beatmungsgerät |
|---|---|
| 2 | Bedienfeld |
| 3 | Anzeige |
| 4 | Kopplung |
| 5 | Verbindungsschlauch |
| 6 | Druckmessschlauch |
| 7 | Druckeingangsstutzen |
| 8 | Schnittstelle |
| 9 | Ausatmungselement |
| 10 | Beatmungsmaske |
| 11 | Kopfhaube |
| 12 | Kopplungselement |
| 13 | Elektromotor mit Lüfterrad |
| 14 | Anwenderschnittstelle (User-Interface) |
| 15 | Motorsteuerung |
| 19 | Sensoreinrichtung |
| 19a | Fluss-Sensor |
| 19b | Druck-Sensor |
| 20 | Patienteninterfacesystem |
| 21 | Bakterienfilter |
| 22 | Anfeuchter |
| 23 | Gänsegurgel |
| 25 | Atemgasstrom |
| 26 | Messwerte |
| 27 | Ausgabeeinheit |
| 30 | Datenauswertungseinrichtung |
| 40 | Alarmgeber |
| 50 | Zeitraum |

60 Grenzwert
70 Steuerung
80 Bewegungsgleichung

**Patentansprüche**

1. Vorrichtung zur Überwachung der Diskonnektion eines Patienteninterfacesystems (20) während der Beatmung mit einem Beatmungsgerät (1), einer Steuerung (70) und einer Einrichtung zur Datenauswertung (30), und einer Einrichtung (19) zur Ermittlung von Werten, die indikativ für den zeitlichen Verlauf des Atemgasstromes (25) sind, deren Ausgangssignale in die Einrichtung zur Datenauswertung (30) eingespeist werden, die wiederum einen Alarmgeber (40) steuert, wobei die Datenauswertungseinrichtung (30) einen Alarm an dem Alarmgeber (40) auslöst, wenn zumindest ein Wert der indikativ für den zeitlichen Verlauf des Atemgasstromes ist für einen bestimmten Zeitraum (50) von einem bestimmten Grenzwert (60) abweicht, wobei die Einrichtung (19) einen Fluss-Sensor (19a) und einen Druck-Sensor (19b) umfasst, wobei die Steuerung (70) derart konfiguriert ist, dass die Einstellung der Grenzwerte (60) automatisch anhand von Daten des Patienteninterface-Systems (20) erfolgt, wobei das Atemgas vom Beatmungsgerät (1) mit angeschlossenem Patienteninterface-System (20) frei, also ohne Patienten, abströmt und das Beatmungsgerät (1) die dabei ermittelten Druck und Flusswerte speichert und als Grenzwerte (60) für die Ermittlung einer Diskonnektion berücksichtigt, und wobei die Grenzwerte Compliancewerte sind.

2. Vorrichtung gemäß Anspruch 1, **dadurch gekennzeichnet, dass** der Zeitraum (50) und/oder der Grenzwert (60) einstellbar sind.

3. Vorrichtung gemäß Anspruch 1 oder 2, **dadurch gekennzeichnet, dass** der Zeitraum (50) und/oder der Grenzwert (60) im Gerät hinterlegt sind oder fortlaufend ermittelt werden.

4. Vorrichtung gemäß Anspruch 2 oder 3, **dadurch gekennzeichnet, dass** der Grenzwert (60) unter Anwendung der Bewegungsgleichung (80) oder davon abgeleiteter Gleichungen und unter Berücksichtigung von Werten, die indikativ für den zeitlichen Verlauf des Atemgasstromes (25) sind, ermittelt wird.

5. Vorrichtung nach zumindest einem der vorhergehenden Ansprüche, **dadurch gekennzeichnet, dass** zudem eine Ausgabeeinheit (22) zur Darstellung der Messwerte (21) vorgesehen ist.

6. Verfahren zur Überwachung der Diskonnektion eines Patienteninterfacesystems während der Beatmung, mittels einer Vorrichtung gemäß einem der Ansprüche 1-5, bei dem Werte die indikativ für den zeitlichen Verlauf des Atemgasstromes sind ermittelt werden und einer Datenauswertung unterzogen werden, wobei basierend auf der Datenauswertung ein Alarm auslöst wird, wenn zumindest ein Wert der indikativ für den zeitlichen Verlauf des Atemgasstromes ist für einen bestimmten Zeitraum von einem bestimmten Grenzwert abweicht.

7. Verfahren nach Anspruch 6 **dadurch gekennzeichnet, dass** der Zeitraum und/oder der Grenzwert einstellbar sind.

8. Verfahren nach zumindest einem der Ansprüche 6-7, **dadurch gekennzeichnet, dass** der Zeitraum und/oder der Grenzwert im Gerät hinterlegt sind oder fortlaufend ermittelt werden.

9. Verfahren nach zumindest einem der Ansprüche 6-8, **dadurch gekennzeichnet, dass** der Grenzwert unter Anwendung der Bewegungsgleichung oder davon abgeleiteter Gleichungen und unter Berücksichtigung von Werten, die indikativ für den zeitlichen Verlauf des Atemgasstromes sind, ermittelt wird.

10. Verfahren nach zumindest einem der Ansprüche 6-9 **dadurch gekennzeichnet, dass** die Einstellung der Werte Zeitraum und/oder Grenzwert automatisch beispielsweise anhand von Daten des Patienteninterfacesystems und/oder über eine Anwenderschnittstelle durch den Anwender erfolgt.

11. Verfahren nach zumindest einem der Ansprüche 6-10 **dadurch gekennzeichnet, dass** zumindest ein Messwert angezeigt wird und über die Anwenderschnittstelle zumindest ein Grenzwert für zumindest einen Messwert des Atemgasstromes und eine Zeitdauer für den Grenzwert vorgebbar ist.

12. Verfahren nach zumindest einem der Ansprüche 6-11 **dadurch gekennzeichnet, dass** durch Anwendung der Bewegungsgleichung Werte ermittelt werden, die indikativ für den zeitlichen Verlauf des Atemgasstromes sind und bei einer Abweichung gegenüber eines bestimmten Zeitraum von einem bestimmten Grenzwert auf eine Diskonnektion geschlossen wird.

**Claims**

1. A device for monitoring the disconnection of a patient interface system (20) during ventilation with a ventilator (1), a control system (70) and an apparatus for data evaluation (30), and an apparatus (19) for establishing values which are indicative of the time curve of the respiratory gas flow (25), the output signals of which are fed into the apparatus for data evaluation (30) which, in turn, controls an alarm transmitter (40), wherein the data evaluation apparatus (30) triggers an alarm on the alarm transmitter (40) if at least one value which is indicative of the time curve of the respiratory gas flow deviates from a specific limit value (60) for a specific period of time (50), wherein the apparatus (19) comprises a flow sensor (19a) and a pressure sensor (19b), wherein the control system (70) is configured in such a way that the limit values (60) are adjusted automatically on the basis of data from the patient interface system (20), wherein the breathing gas freely escapes from the respirator (1) with the connected patient interface system (20), that is to say without patients, and the respirator (1) stores the established pressure and flow values and takes these into account as limit values (60) for the establishment of a disconnection, and wherein the limit values are compliance values.

2. The device according to Claim 1, **characterized in that** the period of time (50) and/or the limit value (60) are adjustable.

3. The device according to Claim 1 or 2, **characterized in that** the period of time (50) and/or the limit value (60) are stored in the ventilator or established continuously.

4. The device according to Claim 2 or 3, **characterized in that** the limit value (60) is established under application of the movement equation (80), or equations derived therefrom, and taking into account values which are indicative of the time curve of the respiratory gas flow (25).

5. The device according to at least one of the preceding claims, **characterized in that** an output unit (22) is additionally provided for displaying the measurement values (21).

6. A method for monitoring the disconnection of a patient interface system during the ventilation, by means of a device according to any one of Claims 1-5, in which values which are indicative of the time curve of the respiratory gas flow are established and subjected to a data evaluation, wherein an alarm is triggered on the basis of the data evaluation if at least one value which is indicative of the time curve of the respiratory gas flow deviates from a specific limit value for a specific period of time.

7. The method according to Claim 6, **characterized in that** the period of time and/or the limit value are adjustable.

8. The method according to at least one of the claims 6-7, **characterized in that** the period of time and/or the limit value is/are stored in the ventilator or established continuously.

9. The method according to at least one of the claims 6-8, **characterized in that** the limit value is established under application of the movement equation or equations derived therefrom, and taking into account values which are indicative of the time curve of the respiratory gas flow.

10. The method according to at least one of the claims 6-9, **characterized in that** the period of time and/or limit value values are adjusted automatically, for example on the basis of data from the patient interface system and/or by the user via a user interface.

11. The method according to at least one of the claims 6-10, **characterized in that** at least one measurement value is displayed, and at least one limit value for at least one measurement value of the respiratory gas flow and a time duration for the limit value can be predetermined by way of the user interface.

12. The method according to at least one of the claims 6-11, **characterized in that** values which are indicative of the time curve of the respiratory gas flow are established, by applying the movement equation and a disconnection is deduced in the event of a deviation from a specific limit value with respect to a specific period of time.

**Revendications**

1. Dispositif de surveillance de la déconnexion d'un système d'interface patient (20) durant la ventilation avec un appareil respiratoire (1), une commande (70) et un système d'analyse de données (30), et avec un système (19) pour déterminer des valeurs, qui sont indicatives de l'évolution temporelle du flux de gaz respiratoire (25), dont les signaux de sortie sont transmis au système d'analyse de données (30), qui commande à son tour un avertisseur (40), dans lequel le système d'analyse de données (30) déclenche une alarme sur l'avertisseur (40), si au moins une valeur indicative de l'évolution temporelle du flux de gaz respiratoire diverge d'une valeur limite définie (60) durant une période définie (50), dans lequel le système (19) comprend un capteur de débit (19a) et un capteur de pression (19b), dans lequel la commande (70) est configurée de telle sorte que le réglage des valeurs limites (60) s'effectue automatiquement à l'aide des données du système d'interface patient (20), dans lequel le gaz respiratoire s'écoule librement, donc sans patient, de l'appareil respiratoire (1) doté du système d'interface patient raccordé (20) et l'appareil respiratoire (1) mémorise la pression et les valeurs de débit alors déterminées et les prend en compte comme des valeurs limites (60) pour déterminer une déconnexion, et dans lequel les valeurs limites sont des valeurs d'observance thérapeutique (*compliance*).

2. Dispositif selon la revendication 1, **caractérisé en ce que** la période (50) et/ou la valeur limite (60) sont réglables.

3. Dispositif selon la revendication 1 ou 2, **caractérisé en ce que** la période (50) et/ou la valeur limite (60) sont enregistrées dans l'appareil ou déterminées en permanence.

4. Dispositif selon la revendication 2 ou 3, **caractérisé en ce que** la valeur limite (60) est déterminée par le biais de l'équation de mouvement (80) ou des équations en dérivant et en fonction des valeurs, qui sont indicatives de l'évolution temporelle du flux de gaz respiratoire (25).

5. Dispositif selon l'une au moins des revendications précédentes, **caractérisé en ce qu'**une unité de sortie (22) est en outre prévue pour représenter les valeurs de mesure (21).

6. Procédé de surveillance de la déconnexion d'un système d'interface patient durant la ventilation, au moyen d'un dispositif selon l'une des revendications 1-5, sur lequel des valeurs, qui sont indicatives de l'évolution temporelle du flux de gaz respiratoire, sont déterminées et soumises à une analyse de données, dans lequel une alarme est déclenchée sur la base de l'analyse de données, si au moins une valeur indicative de l'évolution temporelle du flux de gaz respiratoire diverge d'une valeur limite définie durant une période définie.

7. Procédé selon la revendication 6 **caractérisé en ce que** la période et/ou la valeur limite sont réglables.

8. Procédé selon l'une au moins des revendications 6-7, **caractérisé en ce que** la période et/ou la valeur limite sont enregistrées dans l'appareil ou déterminées en permanence.

9. Procédé selon l'une au moins des revendications 6-8, **caractérisé en ce que** la valeur limite est déterminée par le biais de l'équation de mouvement ou des équations en dérivant et en fonction des valeurs, qui sont indicatives de l'évolution temporelle du flux de gaz respiratoire.

10. Procédé selon l'une au moins des revendications 6-9, **caractérisé en ce que** le réglage des valeurs que sont la période et/ou la valeur limite est réalisé automatiquement par exemple à l'aide des données du système d'interface patient et/ou via une interface utilisateur par l'utilisateur.

11. Procédé selon l'une au moins des revendications 6-10, **caractérisé en ce qu'**au moins une valeur de mesure s'affiche et qu'au moins une valeur limite applicable à au moins une valeur mesurée du flux de gaz respiratoire et une durée applicable à la valeur limite peuvent être prédéfinies via l'interface utilisateur.

12. Procédé selon l'une au moins des revendications 6-10, **caractérisé en ce que** des valeurs, qui sont indicatives de l'évolution temporelle du flux de gaz respiratoire, sont déterminées par le biais de l'équation de mouvement et qu'une déconnexion est présumée si une valeur diverge d'une valeur limite définie par rapport à une période définie.

Fig. 1

Fig. 2

**EP 3 156 091 B1**

**IN DER BESCHREIBUNG AUFGEFÜHRTE DOKUMENTE**

**In der Beschreibung aufgeführte Patentdokumente**

- US 20040016431 A1
- US 20060086357 A1
- EP 0968020 A1
- WO 2014030098 A1
- US 2015144130 A1
- US 20100071696 A1

**In der Beschreibung aufgeführte Nicht-Patentliteratur**

- **UHL RR ; LEWIS FJ.** Digital computer calculation of human pulmonary mechanics using a least squares fit technique. *Comput Biomed Res.,* Oktober 1974, vol. 7 (5), 489-95
- **GUTTMANN J ; EBERHARD L ; FABRY B.** u. a. Determination of volume-dependent respiratory system mechanics in mechanically ventilated patients using the new SLICE method. *Technol Health Care,* 1994, vol. 2, 175-91